Europäisches Patentamt

**European Patent Office**

**Office européen des brevets**

(19)

(11) Veröffentlichungsnummer : **0 118 625**
**B1**

(12) **EUROPÄISCHE PATENTSCHRIFT**

(45) Veröffentlichungstag der Patentschrift :
15.03.89

(51) Int. Cl.⁴ : **A 61 L 9/04, A 61 L 9/01**

(21) Anmeldenummer : **83113258.4**

(22) Anmeldetag : **31.12.83**

(54) Verwendung von Hexamethylcyclotrisiloxan als Riechstoffträgermaterial.

(30) Priorität : **11.02.83 DE 3304822**
**30.03.83 DE 3311642**

(43) Veröffentlichungstag der Anmeldung :
**19.09.84 Patentblatt 84/38**

(45) Bekanntmachung des Hinweises auf die Patenterteilung : **15.03.89 Patentblatt 89/11**

(84) Benannte Vertragsstaaten :
**AT CH DE FR GB IT LI NL SE**

(56) Entgegenhaltungen :
**FR–A– 2 229 753**
**CHEMICAL ABSTRACTS, Band 91, Nr. 24, 10. Dezember 1979, Seiten 363-364, Nr. 198797k, Columbus, Ohio, USA; & JP - A - 79 46847 (SHISEIDO CO., LTD.) 13.04.1979**

(73) Patentinhaber : **Consortium für elektrochemische Industrie GmbH**
**Zielstattstrasse 20**
**D-8000 München 70 (DE)**

(72) Erfinder : **Kreuzer, Franz-Heinrich, Dr. Dipl.-Chem.**
**Josef-Gerstner-Strasse 14d**
**D-8033 Martinsried (DE)**
Erfinder : **Gebauer, Helmut, Dr. Dipl.-Chem.**
**Schaffhauser Strasse 18/7**
**D-8000 München 71 (DE)**

## Beschreibung

Die Erfindung betrifft die Verwendung von Hexamethylcyclotrisiloxan als Riechstoffträgermaterial für Raumbedufter, Toilettenspüleinsätze oder Toilettenkugeln.

Aus der DE-AS 10 63 309 ist die Verwendung von elastischem, kalt verformbaren Methylphenylpolysiloxan als Einbettungsmasse für Riechstoffe bekannt. Die bekannte Polysiloxanmasse dient dazu, die Abgabe des Riechstoffs über einen längeren Zeitraum auszudehnen. Die bekannte Einbettungsmasse hat jedoch den Nachteil, daß sie nicht verdampft, sondern nach der Abgabe des Riechstoffs zurückbleibt und somit das Ende der Riechstoffabgabe nicht oder nur sehr schwer erkennbar ist.

Der Erfindung lag die Aufgabe zugrunde, ein Riechstoffträgermaterial zur Verfügung zu stellen, das bei Raumtemperatur möglichst weitgehend verdampft, um dem Verbraucher das Ende der Riechstoffabgabe erkennbar zu machen. Eine weitere Aufgabe ist es, ein physiologisch unbedenkliches Riechstoffträgermaterial zur Verfügung zu stellen, das keinerlei Geruchsbelästigung verursacht. Eine weitere Aufgabe ist es, ein Riechstoffträgermaterial zur Verfügung zu stellen, das versprühbar ist oder in Wasser schwimmfähig ist.

Die Aufgabe wird gelöst durch die Verwendung von Hexamethylcyclotrisiloxan und gegebenenfalls weiteren verdampfbaren oder nicht verdampfbaren Zusätzen zur Verringerung der Sublimationsgeschwindigkeit des Hexamethylcyclotrisiloxans und gegebenenfalls weiteren bekannten Hilfsstoffen als Riechstoffträgermaterial bei Raumbeduftern Toilettenspüleinsetzen oder Toilettenkugeln.

Aus z. B. Chemical Abstracts, Vol. 91, 1979, Referat 198797 k war die Kombination von Riechstoff mit Hexamethylcyclotrisiloxan lediglich in der Kosmetik bekannt. Raumbedufter, Toilettenspüleinsätze und Toilettenkugeln sind jedoch z. B., schon weil sie im Gegensatz zu Kosmetika nicht in Berührung mit menschlichen Körperteilen kommen, mit Kosmetika nicht allzu nahe verwandt, sodaß vorstehend genannte Druckschrift den Gegenstand der Erfindung nicht nahelegen konnte.

Hexamethylcyclotrisiloxan weist einen Schmelzpunkt von etwa 64 bis 66 °C auf und verdampft bei Raumtemperatur relativ schnell.

Das erfindungsgemäße Riechstoffträgermaterial wird als Raumbedufter, Toilettenspüleinsatz, für Toilettenkugeln, Auftriebskörper in Toilettenspülkästen, als sprühfähige Aerosolmischung usw. verwendet.

Geeignete Riechstoffe für das erfindungsgemäße Trägermaterial sind Kohlenwasserstoffe, Resinoide, Absolue und/oder natürliche und synthetische Parfümöle insbesondere solche, die z. B. als funktionelle Gruppen Hydroxyl-, Äther-, Ester-, Aldehyd-, Keto-, Säure-, Nitril-, Nitro-Gruppen, Chloratome enthalten oder eine Terpen- oder Sesquiterpen-Gruppe, z. B. Anethol, Anisaldehyd, Vanillin oder Citronellal. Als geeignete Riechstoffe sind auch N-haltige organische Verbindungen und aliphatische und aromatische Kohlenwasserstoffe mit gesättigten und/oder ungesättigten Substituenten einsetzbar. Geeignete ätherische Öle sind z. B. Anisöl, Bergamotteöl, Campheröl, Citronellöl, Citronenöl, Eucalyptusöl, Fichtennadelöle, Geraniumöl, Lavendelöl, Lemongrasöl, Nelkenöl, Orangenöl, Pomeranzenöl, Pfefferminzöl, Rosenöl, Spiköl, Terpentinöl und Zimtöl.

Die Menge an Hexamethylcyclotrisiloxan beträgt 0,5 bis 98 Gew.-%, insbesondere 2 bis 60 Gew.-%, bezogen auf das Gesamtgewicht der jeweiligen Raumbedufter Toilettenspüleinsätze bzw. Toilettenkugeln. Die Menge des Riechstoffs bzw. der Riechstoffkomposition beträgt 2 bis 50 Gew.-%, insbesondere 3 bis 12 Gew.-%, bezogen auf das Gesamtgewicht der jeweiligen Raumbedufter, Toilettenspüleinsätze bzw. Toilettenkugeln.

Das erfindungsgemäß eingesetzte Trägermaterial kann Stabilisatoren enthalten, die eine Polymerisation der verdampfbaren Siloxanverbindung zu nicht verdampfbaren, hochpolymeren Siloxanverbindungen verhindert bzw. verzögert. Auf diese Weise kann eine eventuelle Beeinträchtigung der Verdampfungsgeschwindigkeit der erfindungsgemäßen Trägermaterialien, die bei einer längeren Lagerung eintreten kann, weitgehend verhindert werden. Als Stabilisatoren für die Hexamethylcyclotrisiloxane sind z. B. Erdalkalimetalloxide, insbesondere MgO, BaO und/oder CaO, geeignet. Die Menge des gegebenenfalls zugesetzten Stabilisators beträgt 0,1 bis 1 Gew.-%, bezogen auf das Gewicht des Hexamethylcyclotrisiloxans.

Die Sublimationsgeschwindigkeit des Hexamethylcyclotrisiloxans macht sich vorteilhafterweise dann bemerkbar, wenn das Siloxan als Lift für das Parfumöl verwendet wird, d. h. wenn der Geruchsverbesserer nur aus dem Siloxan und dem Parfumöl besteht und wenn das Siloxan in kleiner Menge eingesetzt wird, z. B. zu 0,5 bis 30 %, bezogen auf das Gesamtgewicht aus Parfumöl und Siloxan.

Die Sublimationsgeschwindigkeit des Hexamethylcyclotrisiloxans ist für einige Anwendungsgebiete zu hoch. Dem Trägermaterial werden daher Zusatzstoffe zugegeben, die die Sublimationsgeschwindigkeit des Siloxans herabsetzen. Der Geruchsverbesserer kann aber auch mit Zusatzstoffen umhüllt werden, die nach dem Auftragen bei Raumtemperatur erstarren und die die Sublimationsgeschwindigkeit herabsetzen. Geeignete Zusatzstoffe sind z. B. Paraffin, Stearin, Paraffinöl, Ester von Harzsäuren, Polyamidharze, Ethylcellulose, Vinylacetat-Vinylchlorid-Mischpolimerisate, Polyvinylalkohol, Gelatine, Stärke, Epoxidharze, Polychloroprene, Polyisobutylen, Kampfer, Naphthalin, Tetramethylcyclobutandion, Trialkyltrioxan, Klebstoffe, Camphen, Tricyclodecan, Montanharz, Paraffinwachs, Montanwachs, Polyvinylacetat, Polyvinylpyrrolidon, Calciumcarbonat, Ton, Seife, Aluminiumoxid, Resinoide, Wasserglas, Silikate und/oder Kieselerde.

Die Zusatzstoffe werden, sofern es sich um Feststoffe handelt, in sehr fein verteilter Form zum Duftstoffträger zugemischt, die Teilchengröße der Zusätze ist nicht kritisch. Sie liegt aber vorzugsweise bei 0,5 mm und darunter. Die festen, nicht verdampfbaren Zusatzstoffe sind möglichst homogen im Geruchsverbesserer verteilt und liegen nur in einer geringen Menge vor, so daß sie beim Verdampfen des Duftstoffträgers und des Riechstoffs in gleichmäßiger fein verteilter Form anfallen.

Der Zusatzstoff kann auch aus einem Feststoff bestehen, der dem Geruchsverbesserer in Wasser einen Auftrieb verleiht, z. B. Schaumstoffteilchen aus Polyurethan oder Polystyrol. Diese Geruchsverbesserer sind schwimmfähig und werden daher bevorzugt für die Wasserreservoirbehälter von Toilettenspülanlagen verwendet. Daneben enthalten diese Geruchsverbesserer vorzugsweise noch Tenside und/oder Desinfektionsmittel.

Die Zusatzstoffe können sowohl mit dem Siloxanträgermaterial vermischt und/oder als dampfdurchlässige Hülle auf das Riechstoffträgermaterial aufgebracht werden.

Die in Wasser und/oder einem Lösungsmittel löslichen Zusatzstoffe können in Form der Lösung auf das Siloxanträgermaterial aufgebracht werden. Dies kann z. B. durch Eintauchen des Geruchsverbesserers in eine Lösung oder durch das Aufsprühen der Lösung auf die Oberfläche des Geruchsverbesserers vorgenommen werden. Nach dem Aufbringen der Lösung wird das Lösungsmittel durch Verdampfen abgezogen. Der Vorgang des Eintauchens bzw. Besprühens kann auch mehrfach erfolgen. Die Sublimation des Siloxanträgermaterials darf jedoch nicht völlig verhindert werden.

Das Aufbringen des Zusatzstoffes auf die Oberfläche des Geruchsverbesserers, bestehend aus dem Hexamethylcyclotrisiloxan, Riechstoff und gegebenenfalls weiteren Hilfsstoffen, kann auch durch Eintauchen in eine Schmelze des Zusatzstoffes vorgenommen werden. Die Dicke des Überzugs aus der Schmelze des Zusatzstoffes kann z. B. durch mehrfaches Eintauchen in die Schmelze so eingestellt werden, bis die gewünschte Sublimationsgeschwindigkeit des Siloxanträgermaterials erreicht ist. Auf diese Weise kann die gewünschte Sublimationsrate anhand einfacher Laborversuche auf den jeweiligen Anwendungsfall eingestellt werden. Auch das Ausmaß der Wasserlöslichkeit eines wasserlösliche Substanzen enthaltenden Geruchsverbesserers kann durch die Art und Dicke des Überzugs in gewünschter Weise beeinflußt werden.

Die flüssigen und/oder festen Zusatzstoffe werden im allgemeinen in einer Menge von 6 bis 50 Gew.-%, insbesondere 10 bis 40 Gew.-%, bezogen auf das Gesamtgewicht des Geruchsverbesserers, zugesetzt.

Gemäß einer Ausgestaltung der Erfindung kann der Geruchsverbesserer auch aus einem Treibgas, Hexamethylcyclotrisiloxan und einem Parfumöl bestehen. Als Treibgas werden z. B. ein Fluorkohlenwasserstoff, Propan oder Butan eingesetzt. Das Parfumöl bzw. die Riechstoffkomposition kann auch ersetzt werden durch ein Desinfektionsmittel, sofern das Desinfektionsmittel keinen unangenehmen Geruch besitzt.

Die Aerosolmischung kann gegebenenfalls neben dem Parfumöl auch Desinfektionsmittel zusätzlich enthalten. Das Aerosolgemisch wird unter Druck in Sprühvorrichtungen eingefüllt. Das Aerosolgemisch kann als weißes trockenes Pulver ausgesprüht werden, das rückstandslos verdampft, und zwar in Abhängigkeit von der ausgesprühten Menge. Die Aerosolmischungen eignen sich insbesondere als Raumbedufter, Kleiderspray, Möbelspray oder auch als Christbaumspray zur Erzeugung von künstlichem Schnee. Der Vorteil des Sprays liegt darin, daß der Duft hinsichtlich der Stärke und der Zeitdauer exakt dosiert werden kann und daß der Verbraucher sofort erkennt, wenn die Duftquelle verbraucht ist, und zwar dadurch, daß das ausgesprühte Material verdampft ist. Bei WC-Desinfektionssprays kann der Parfumölzusatz relativ gering gehalten werden oder er kann auch ganz weggelassen werden. Die Menge des Hexamethylcyclotrisiloxans in den Aerosolmischungen liegt vorzugsweise bei etwa 20 bis 60 Gew.-%, bezogen auf das Gesamtgewicht der flüssigen Aerosolmischung.

Es kann von Vorteil sein, daß der Duftstoffträger für spezielle Anwendungsgebiete, z. B. wenn er als Toilettenkugel verwendet wird, Waschhilfsstoffe, Tenside und/oder Desinfektionsmittel enthält. Als Tenside können alle an sich bekannten Tenside verwendet werden, z. B. anionaktive, kationaktive, nichtionogene und amphotere Tenside, wie Seifen, Sulfonate, Aminsalze, Invertseifen, quartäre Ammoniumverbindungen, Äthoxylate, Aminoxide und/oder Betaine.

Als Waschhilfsstoffe kommen z. B. Harnstoff, Natriumsulfat und/oder Natriumcarbonat in Frage.

Als Desinfektionsmittel werden insbesondere Grobdesinfektionsmittel eingesetzt, die zur Bekämpfung von pathogenen Mikroorganismen geeignet sind, z. B. Chlorate, Hypochlorite, Chlorkalk, Chloramine, Methyl- und/oder Chlorderivate des Phenols, Chinolin, Acridin, quartäre Ammoniumverbindungen, Amphotenside und Gemische davon.

Die Tenside und Waschhilfsstoffe dienen zur gleichzeitigen Reinigung der Toilettenbecken, in denen die erfindungsgemäßen Geruchsverbesserer angeordnet werden. Die Menge dieser Hilfsstoffe (Tenside, Waschhilfsstoffe und/oder Desinfektionsmittel) beträgt 3 bis 15 Gew.-%, insbesondere 5 bis 10 Gew.-%, entweder als Einzelverbindung oder als Gemisch, bezogen auf das Gesamtgewicht des Geruchsverbesserers.

Die erfindungsgemäßen Geruchsverbesserer können hergestellt werden, indem man Hexamethylcyclotrisiloxan mit der Riechstoffkomponente bzw. der Riechstoffkomposition homogen vermischt und gegebenenfalls die Zusätze hinzugibt und die Mischung dann zu einem Duftstoffträgerkörper verpreßt unter Druckanwendung und gegebenenfalls unter zusätzlicher Wärmeeinwirkung. Das Verpressen der Bestandteile des Duftstoffträgers kann z. B. durch übliche Pressen, die zur Herstellung von Tabletten oder Kugeln oder Kapseln geeignet sind, vorgenommen werden.

Eine besonders homogene und dichte Packungsform wird erreicht, wenn die Bestandtei-le des Duftstoffträgermaterials durch Verschmel-zen miteinander vermischt und dann abgekühlt werden.

Die Verringerung der Sublimationsgeschwin-digkeit des erfindungsgemäß eingesetzten Silo-xans kann nach einer vorteilhaften Ausgestaltung der Erfindung auch dadurch erreicht werden, daß die körperlichen Duftstoffträgermaterialien mit einem die Sublimation herabsetzenden Überzug versehen werden, insbesondere bestehend aus Polyvinylalkohol, Polyvinylpyrrolidon, Montanwachs, Montanharz und/oder Tricyclodecan. Die Preßlinge aus Hexamethylcyclotrisiloxan und Riechstoff werden vorzugsweise in ein Tauchbad, enthaltend den sublimationshemmenden Zusatzstoff als Schmelze oder Lösung, eingetaucht, um den Überzug aufzubringen. Ein besonders geeignetes Tauchbad besteht z. B. aus einer Lösung von Polyvinylacetat und/oder Polypyrrolidon in Alkylalkohol, wie Methanol, Ethanol, Isopropanol. Dabei werden insbesondere Zusatzstoffe als Über-zugsmaterialien verwendet, die ebenfalls subli-mieren, aber eine geringere Sublimationsge-schwindigkeit aufweisen als Hexamethylcyclotri-siloxan. Für diesen Zweck sind insbesondere Campher, Champhen, Tricyclodecan und/oder Tetramethylcyclobutandion geeignet.

Daß wie oben bereits angegeben, das Hexamethylcyclotrisiloxan als Lift für den verwendeten Riechstoff wirkt, macht sich bei Riechstoffen mit geringem Dampfdruck besonders vorteilhaft bemerkbar.

Nach einer besonderen Ausgestaltung der Erfindung können auch Zusatzstoffe verwendet werden, die eine eigene Riechstoffwirkung besitzen, z. B. Campher, Camphen, Tricyclodecan und einige Desinfektionsmittel. In diesem Fall kann auf den Zusatz eines Parfümöls verzichtet werden, falls der Anwendungszweck dies zuläßt.

Besonders bevorzugt als Zusatzstoff für die Herabsetzung der Sublimationsgeschwindigkeit des Hexamethylcyclotrisiloxans ist das Trialkyltrioxan. Besonders bevorzugt ist das Tri($C_6$-$C_3$-alkyl)-trioxan, insbesondere das Triisopropyltrioxan und Tri-t-butyltrioxan.

Die Erfindung wird durch die folgenden Beispiele näher erläutert :

Beispiel 1

4,75 g Hexamethylcyclotrisiloxan wurden mit 0,25 g Terpineol vermischt und dann zu einer lose gepackten Tablette verpreßt. Beim Stehenlassen der Tablette bei Raumtemperatur waren nach 5 Tagen 50 % (2,5 g) des Duftstoffkörpers ver-dampft.

Beispiel 2

Es wurden 20 g Hexamethylcyclotrisiloxan in 80 g der Riechstoffkomposition « drom C » aufgelöst. Die Riechstoffkomposition « drom C » besteht aus

10 g Citronellal
40 g Citronellaöl Java
9 g Citronellol
1 g sog. Aldehyd 16
10 g Benzylacetat
20 g Litcea-cubebaöl
10 g Terpineol

Eingearbeitet in Seifen oder wenn man Zellstoff mit der Lösung tränkt, ergibt diese Mischung einen intensiveren Geruch als bei Verwendung der Riechstoffkomposition ohne das Siloxan. Das Siloxan wirkt somit als sog. Lift für den Riechstoff.

Beispiel 3

3,5 g Hexamethylcyclotrisiloxan, 2 g Paraffinöl und 0,5 g Terpineol wurden miteinander vermischt und dann zu einem kugelförmigen Riechstoffträger (lose gepacktes Pulver) verpreßt. Beim Stehenlassen der Probe beim Raumtemperatur waren nach 25 Tagen 2 g (33,3 %) des Duftstoffkörpers verdampft.

Beispiel 4

Es wurden 2,5 g Hexamethylcyclotrisiloxan, 2 g Kampfer und 0,5 g der Riechstoffkomposition « drom C » vermischt und anschließend zu einer lose gepackten Tablette verpreßt. Die Riechstoffkomposition « drom C » ist identisch mit der in Beispiel 2 beschriebenen Zusammensetzung.

Die Probe wurde bei Raumtemperatur gelagert und dann die Sublimationsgeschwindigkeit bestimmt. Nach einer Lagerung von 14 Tagen waren insgesamt 3 g des Duftstoffkörpers verdampft.

Beispiel 5

Es wurde eine Probe hergestellt aus 3 g Hexamethylcyclotrisiloxan, 0,6 g der Riechstoffkomposition « drom C » und 2,4 g Paraffinwachs als sublimationshemmenden Zusatzstoff. Nach ca. 15 Tagen waren 2,8 g des Duftstoffkörpers verdampft.

Beispiel 6

Es wurde eine Probe hergestellt aus 3 g Hexa-methylcyclotrisiloxan, 0,6 g Riechstoffkomposi-tion « drom C » und 2,4 g Polyethylenglycol mit einem Molekulargewicht von 1500 bis 4000 als Zusatzstoff. Nach 14 Tagen waren ca. 2,5 g der Substanzen verdampft.

Beispiel 7

Es wurden 5 g Hexamethylcyclotrisiloxan, 3 g Ethylcellulose und 1 g Terpineol vermischt und das so hergestellte Pulver hinsichtlich der Sublimationsgeschwindigkeit untersucht. Es wurde festgestellt, daß bei Lagerung bei Raumtemperatur nach 16 Tagen 3,5 g des Pulvers verdampft waren.

Beispiel 8

5 g Hexamethylcyclotrisiloxan wurden mit 0,5 g Terpineol vermischt und zu einer Tablette verpreßt. Die so hergestellte Tablette wurde dann mit einem Polyurethanlack überzogen. Der Überzug kann durch Besprühen oder durch Eintauchen in ein Polyurethan-Lackbad hergestellt werden. Die Verdampfungsrate des Siloxans wurde durch den Überzug erheblich herabgesetzt.

Beispiel 9

Es wurden 3,5 g Hexamethylcyclotrisiloxan mit 2 g Paraffinöl und 0,5 g Terpineol vermischt und zu einer flachen, linsenförmigen Tablette verpreßt. Danach wurde die so hergestellte Tablette mit einer dampfdurchlässigen Kunststoffolie auf beiden Seiten überzogen. Beim Lagern der so präparierten Tablette bei Raumtemperatur wurde eine erhebliche Verringerung der Sublimationsgeschwindigkeit gegenüber der nicht beschichteten Tablette festgestellt. Nach 25 Tagen waren bei Raumtemperatur nur 0,5 g des Duftstoffkörpers verdampft.

Beispiel 10

Es wurden 6,2 g Hexamethylcyclotrisiloxan mit 0,3 g Kieselgel, 0,5 g Paraffinwachs, 2 g Campher und 1 g Camphen homogen gemischt und in einer Handpresse zu einem tablettenförmigen, festen Riechstoffkörper verpreßt. Die Verdampfungsrate betrug bei Raumtemperatur nach 15 Tagen 6 g, bezogen auf das Gesamtgewicht des Geruchsverbesserers.

Beispiel 11

15 g Hexamethylcyclotrisiloxan, 6 g Tricyclodecan und 1,0 g Terpineol wurden homogen miteinander vermischt und dann mit einer Handpresse zu einem kugelförmigen Riechstoffträger verpreßt. Beim Stehenlassen der Probe bei Raumtemperatur waren nach 17 Tagen 14 g des Duftstoffkörpers verdampft. Die gleiche Probe, jedoch ohne den zusätzlichen Riechstoff Terpineol wies die gleiche Verdampfungsrate auf.

Beispiel 12

Es wurden 15 g Hexamethylcyclotrisiloxan, 4 g Montanwachs und 1,0 g der Riechstoffkomposition « drom C » gemäß Beispiel 4 vermischt und anschließend mit einer Handpresse zu einer lose gepackten Tablette verpreßt.
Die Probe wurde bei Raumtemperatur gelagert und dann die Sublimationsgeschwindigkeit bestimmt. Nach einer Lagerung von 17 Tagen waren insgesamt 13 g des Duftstoffkörpers verdampft.

Beispiel 13

Es wurde eine Geruchsverbesserer-Tablette, bestehend aus 19 g Hexamethylcyclotrisiloxan und 1 g Citronellaöl in eine Lösung von Polyvinylacetat und Polyvinylpyrrolidon in Isopropanol getaucht und anschließend bei Raumtemperatur getrocknet. Nach der Trocknung hatte sich ein gleichmäßiger, dünner Überzug aus Polyvinylacetat/Polyvinylpyrrolidon auf der Oberfläche der Geruchsverbesserer-Tablette gebildet. Die mit der Kunststoffhülle umgebene Tablette wurde bei Raumtemperatur gelagert, um die Verdampfungsrate zu bestimmen. Nach 14 Tagen war der Geruchsverbesserer verdampft, wobei die zusammengefallene Kunststoffhülle zurückblieb.

Der obige Versuch wurde wiederholt, jedoch mit der Ausnahme, daß die Tablette zweimal in die obige Lösung eingetaucht wurde. Es wurde festgestellt, daß die Sublimationsrate dieser Tablette geringer war als die der Probe, die nur einmal in die Polyvinylacetat/Polyvinylpyrrolidon-Lösung getaucht worden war.

Beispiel 14

Es wurden zwei Geruchsverbesserer-Tabletten aus jeweils 19 g Hexamethylcyclotrisiloxan und 1 g Isobornylacetat hergestellt. Die erste Tablette wurde dann einmal in eine Schmelze von Montanwachs getaucht. Nach dem Erstarren des Überzugs wurde die Sublimationsrate bei Raumtemperatur bestimmt. Sie betrug nach 1 Tag 6,8 g, bezogen auf das Gesamtgewicht des Geruchsverbesserers.

Bei der zweiten Tablette wurde das Eintauchen in die Montanwachs-Schmelze dreimal wiederholt, um einen dreifachen Überzug auf die Tablette herzustellen. Die Sublimationsrate betrug bei dieser Probe nur 3,8 g pro Tag.

Beispiel 15

Es wurde ein Aerosol aus den folgenden Bestandteilen hergestellt :

40 % Hexamethylcyclotrisiloxan
4 % Riechstoffkomposition « drom C »
56 % Fluor-Kohlenwasserstoff als Treibgas

Beim Aussprühen des Gemisches aus einem unter Druck stehenden Sprühbehälter bildet sich ein weißer, trockener Schnee, der je nach der ausgesprühten Menge innerhalb von 2 bis 10 h rückstandslos unter Beduftung des Raumes verdampft.

**Patentansprüche**

1. Verwendung von Hexamethylcyclotrisiloxan als Riechstoffträgermaterial bei Raumbeduftern, Toilettenspüleinsätzen oder Toilettenkugeln, gegebenenfalls in Verbindung mit weiteren verdampfbaren oder nicht verdampfbaren Zusätzen, zur Verringerung der Sublimationsgeschwindigkeit des Hexamethylcyclotrisiloxans.

2. Verwendung nach Anspruch 1, dadurch gekennzeichnet, daß desinfizierende Hilfsstoffe oder Waschhilfsstoffe mitverwendet werden.

3. Verwendung nach Anspruch 1, dadurch gekennzeichnet, daß Paraffin, Stearin, Paraffinöl, Ester von Harzsäuren, Polyamidharze, Ethylcellulosen, Vinylacetat-vinylchloridcopolymere, Polyvinylalkohol, Gelatine, Stärke, Epoxyharze, Polychloropren, Polyisobutylen, Kampfer, Naphthalin, Tetramethylcyclobutandion, Trialkyltrioxan, Klebstoffe, Camphen, Tricyclodecan, Montanharz, Paraffinwachs, Montanwachs, Polyvinylacetat, Polyvinylpyrrolidon, Calciumcarbonat, Ton, Aluminiumoxid, Resinoiden, Wasserglas, Silicate und/oder Kieselgel als Zusatzstoff mitverwendet werden.

4. Verwendung nach Anspruch 1, dadurch gekennzeichnet, daß natürliche oder synthetische Parfumöle, Kohlenwasserstoffe, Resinoide und/oder Absolue als Riechstoff mitverwendet werden.

5. Verwendung nach Anspruch 4, dadurch gekennzeichnet, daß das Parfumöl Hydroxyl-, Ether-, Ester-, Aldehyd-, Säure-, Nitril-, Keto- oder Nitro-Gruppen, Chloratome und/oder Terpen-Gruppen als funktionelle Gruppen besitzt.

6. Verwendung nach Anspruch 1, dadurch gekennzeichnet, daß die Zusatzstoffe fest sind und das Riechstoffträgermaterial in Form einer dampfdurchlässigen Hülle umgeben.

7. Verwendung nach Anspruch 1, dadurch gekennzeichnet, daß 0,5 bis 98 Gew.-%, insbesondere 2 bis 60 Gew.-% Hexamethylcyclotrisiloxan, bezogen auf das Gesamtgewicht der jeweiligen Raumbedufter, Toilettenspüleinsätze bzw. Toilettenkugeln eingesetzt werden.

8. Verwendung nach Anspruch 1, dadurch gekennzeichnet, daß 2 bis 50 Gew.-% Riechstoffe und/oder Riechstoffkompositionen, bezogen auf das Gesamtgewicht der jeweiligen Raumbedufter, Toilettenspüleinsätze bzw. Toilettenkugeln eingesetzt werden.

9. Verwendung nach Anspruch 1, dadurch gekennzeichnet, daß 6 bis 50 Gew.-% flüssige und/oder feste Zusatzstoffe, bezogen auf das Gesamtgewicht der jeweiligen Raumbedufter, Toilettenspüleinsätze bzw. Toilettenkugeln eingesetzt werden.

10. Verwendung nach Anspruch 1, dadurch gekennzeichnet, daß 3 bis 15 Gew.-% Desinfektionsmittel, bezogen auf das Gesamtgewicht der jeweiligen Raumbedufter, Toilettenspüleinsätze bzw. Toilettenkugeln eingesetzt werden.

11. Verwendung nach Anspruch 1, dadurch gekennzeichnet, daß 0,1 bis 1 Gew.-% wenigstens eines Erdalkalimetalloxids, bezogen auf das Gewicht des Hexamethylcyclotrisiloxans eingesetzt wird.

## Claims

1. Use of hexamethylcyclotrisiloxane as a scent carrier material in air fresheners, lavatory bowl blocks or urinal tablets, optionally in combination with further volatile or non-volatile additives for reducing the sublimation rate of the hexamethylcyclotrisiloxane.

2. Use according to Claim 1, characterized in that disinfectant auxiliaries or detergent auxiliaries are also used.

3. Use according to Claim 1, characterized in that paraffin, stearin, paraffin oil, esters of resin acids, polyamide resins, ethylcelluloses, vinyl acetate/vinyl chloride copolymers, poly (vinyl alcohol), gelatin, starch, epoxy resins, polychloroprene, polyisobutylene, camphor, naphthalene, tetramethylcyclobutanedione, trialkyltrioxane, adhesives, camphene, tricyclodecane, montan resin, paraffin wax, montan wax, poly (vinyl acetate), polyvinylpyrrolidone, calcium carbonate, clay, aluminium oxide, resinoids, water glass, silicates and/or silica gel are co-used as additive.

4. Use according to Claim 1, characterized in that natural or synthetic perfume oils, hydrocarbons, resinoids and/or absolutes are co-used as scent.

5. Use according to Claim 4, characterized in that the perfume oil contains hydroxyl, ether, ester, aldehyde, acid, nitrile, keto or nitro groups, chlorine atoms and/or terpene groups as functional groups.

6. Use according to Claim 1, characterized in that the additives are solid and coat the scent carrier material in the form of a vapour-permeable coating.

7. Use according to Claim 1, characterized in that 0.5 to 98 % by weight, in particular 2 to 60 % by weight, of hexamethylcyclotrisiloxane, relative to the total weight of the particular air fresheners, lavatory bowl blocks or urinal tablets, are employed.

8. Use according to Claim 1, characterized in that 2 to 50 % by weight of scents and/or scent compositions, relative to the total weight of the particular air fresheners, lavatory bowl blocks or urinal tablets, are employed.

9. Use according to Claim 1, characterized in that 6 to 50 % by weight of liquid and/or solid additives, relative to the total weight of the particular air fresheners, lavatory bowl blocks or urinal tablets, are employed.

10. Use according to Claim 1, characterized in that 3 to 15 % by weight of disinfectant, relative to the total weight of the particular air fresheners, lavatory bowl blocks or urinal tablets are employed.

11. Use according to Claim 1, characterized in that 0.1 to 1 % by weight of at least one alkaline earth metal oxide, relative to the weight of the hexamethylcyclotrisiloxane, is employed.

## Revendications

1. Utilisation de l'hexaméthylcyclotrisiloxane comme support de matières odorantes pour des désodorisants d'ambiance, des garnitures pour l'eau de rinçage des toilettes ou pour des billes ou sphères pour toilettes, éventuellement en liaison ou association avec d'autres additifs évaporables ou non évaporables, pour diminuer la vitesse de sublimation de l'hexaméthylcyclotrisiloxane.

2. Utilisation selon la revendication 1, caractérisée en ce qu'on utilise également des adjuvants désinfectants ou des adjuvants de lavage.

3. Utilisation selon la revendication 1, caractérisée en ce qu'on utilise également, comme additif, de la paraffine, de la stéarine, de l'huile de paraffine, des esters d'acides résiniques, de la résine de polyamide, des éthylcelluloses, des copolymères acétate de vinyle/chlorure de vinyle, du poly(alcool vinylique), des gélatines, de la fécule ou amidon, de la résine époxyde, du polychloroprène, du polyisobutylène, du camphre, du naphtalène, de la tétraméthylcyclobutanedione, un trialkyltrioxane, de la colle ou adhésif, du camphène, du tricyclodécane, de la résine de Montana, de la cire de paraffine, de la cire de Montana, du poly (acétate de vinyle), de la polyvinylpyrrolidone, du carbonate de calcium, de l'argile, de l'oxyde d'aluminium, des résinoïdes, des silicates hydrosolubles (« verre à l'eau »), des silicates et/ou du gel de silice.

4. Utilisation selon la revendication 1, caractérisée en ce qu'on utilise également des essences naturelles ou synthétiques de parfum, des hydrocarbures, des résinoïdes et/ou des extraits comme parfums.

5. Utilisation selon la revendication 4, caractérisée en ce que l'essence de parfum contient, comme groupes fonctionnels, des groupes hydroxyles, éthers-oxydes, esters, aldéhydes, acides, nitriles, cétones ou nitro, des atomes de chlore et/ou des groupes terpéniques.

6. Utilisation selon la revendication 1, caractérisée en ce que les additifs sont solides et entourent, sous forme d'une enveloppe perméable à la vapeur, la matière de support de la matière odorante.

7. Utilisation selon la revendication 1, caractérisée en ce qu'on utilise 0,5 à 98 % en poids, notamment 2 à 60 % en poids, d'hexaméthylcyclotrisiloxane, par rapport au poids total du désodorisant d'ambiance, de la garniture ou additif pour l'eau de rinçage à des toilettes ou pour des billes ou sphères pour toilettes.

8. Utilisation selon la revendication 1, caractérisée en ce qu'on utilise 2 à 50 % en poids de matières odorantes et/ou de compositions de matières odorantes, par rapport au poids total du désodorisant d'ambiance, des additifs ou garnitures pour l'eau de rinçage des toilettes ou pour billes ou sphères pour toilettes.

9. Utilisation selon la revendication 1, caractérisée en ce qu'on utilise 6 à 50 % en poids d'additifs liquides et/ou solides, par rapport au poids total du désodorisant d'ambiance, de l'additif ou de garnitures pour eau de rinçage de toilettes ou de billes ou sphères pour toilettes.

10. Utilisation selon la revendication 1, caractérisée en ce qu'on utilise 3 à 15 % en poids d'un désinfectant, par rapport au poids total du désodorisant d'ambiance, de l'additif ou garniture pour eau de rinçage de toilette ou pour des billes ou sphères de toilettes.

11. Utilisation selon la revendication 1, caractérisée en ce qu'on utilise 0,1 à 1 % en poids d'au moins un oxyde de métal alcalino-terreux, par rapport au poids de l'hexaméthylcyclotrisiloxane.